# EUROPÄISCHE PATENTSCHRIFT

(11) **EP 1 382 684 B1**
(45) Veröffentlichungstag und Bekanntmachung des Hinweises auf die Patenterteilung: **07.12.2005**
(21) Anmeldenummer: 03015546.9
(22) Anmeldetag: 10.07.2003
(51) Int. Cl.: C12P 13/06, C12P 13/12, C12R 1/19

(54) **Verfahren zur fermentativen Herstellung von Aminosäuren und Aminosäure-Derivaten der Phosphoglycerat-Familie**
Process for fermentative production of amino acids and amino acid derivatives of the phosphoglycerate family
Procédé pour la préparation par fermentation d'acide aminés et de ses derivés de la famille phosphoglycerate

(30) Priorität: 19.07.2002 DE 10232930
(43) Veröffentlichungstag der Anmeldung: 21.01.2004
(73) Patentinhaber: Consortium für elektrochemische Industrie GmbH, 81379 München (DE)
(72) Erfinder: Maier, Thomas, Dr., 85221 Dachau (DE)
(74) Vertreter: Potten, Holger

(56) Entgegenhaltungen:
- EP-A- 1 016 710
- NAKAMORI ET AL: "overproduction of L-cysteine and L-cystine by Escherichia coli strains with a genetically altered serine acetyltransferase" APPLIED AND ENVIRONMENTAL MICROBIOLOGY, WASHINGTON,DC, US, Bd. 64, Nr. 5, Mai 1998 (1998-05), Seiten 1607-1611, XP002115630 ISSN: 0099-2240
- NASHIMOTO: "YFIK P38101" EMBL/SWISSPROT, XP002142362
- DASSLER T ET AL: "IDENTIFICATION OF A MAJOR FACILITATOR PROTEIN FROM ESCHERICHIA COLIINVOLVED IN EFFLUX OF METABOLITES OF THE CYSTEINE PATHWAY" MOLECULAR MICROBIOLOGY, BLACKWELL SCIENTIFIC, OXFORD, GB, Bd. 36, Nr. 5, Juni 2000 (2000-06), Seiten 1101-1112, XP000992212 ISSN: 0950-382X
- FRANKE ISABEL ET AL: "YfiK from Escherichia coli promotes export of O-acetylserine and cysteine." JOURNAL OF BACTERIOLOGY, Bd. 185, Nr. 4, 20. Februar 2003 (2003-02-20), Seiten 1161-1166, XP008020355 ISSN: 0021-9193

## Beschreibung

Die Erfindung betrifft ein Verfahren zur Herstellung der Aminosäuren O-Acetyl-L-Serin, N-Acetyl-L-Serin, L-Cystein mittels Fermentation.

Die Herstellung der zwanzig natürlichen, proteinogenen Aminosäuren wird heutzutage vorwiegend durch Fermentation von Mikroorganismen bewerkstelligt. Dabei wird ausgenützt, dass Mikroorganismen über entsprechende Biosynthesewege zur Synthese der natürlichen Aminosäuren verfügen.

Solche Biosynthesewege unterliegen jedoch in Wildtyp-Stämmen einer strengen Kontrolle, die gewährleistet, dass die Aminosäuren nur zum Eigenbedarf der Zelle hergestellt werden. Eine wichtige Voraussetzung für effiziente Produktionsprozesse ist es deshalb, dass geeignete Mikroorganismen verfügbar sind, die im Gegensatz zu Wildtyp-Organismen eine drastisch gesteigerte Produktionsleistung für die Herstellung der gewünschten Aminosäure aufweisen.

Solche Aminosäure-überproduzierende Mikroorganismen können durch klassische Mutations-/Selektionsverfahren und/oder durch moderne, gezielte, rekombinante Techniken ("metabolic engineering") erzeugt werden. Bei letzterem werden zunächst Gene oder Allele identifiziert, die durch ihre Veränderung, Aktivierung oder Inaktivierung eine Überproduktion bewirken. Diese Gene/Allele werden dann durch molekularbiologische Techniken in einen Mikroorganismenstamm eingebracht oder inaktiviert, so dass eine optimale Überproduktion erzielt wird. Häufig führt jedoch erst die Kombination mehrerer, verschiedener Maßnahmen zu einer wirklich effizienten Produktion.

Die Phosphoglycerat-Familie von Aminosäuren ist dadurch definiert, dass es sich um Aminosäuren handelt, die in ihrer Biosynthese von der 3-Phosphoglycerinsäure abgeleitet werden. Der natürliche Pfad des Stoffwechsels führt dabei zunächst über die Zwischenstufen 3-Phospohydroxypyruvat und 3-Phospho-L-serin zu L-Serin. L-Serin kann weiterhin zu Glycin bzw. über O-Acetyl-L-Serin zu L-Cystein umgesetzt werden.

Für die fermentative Herstellung von Aminosäuren der Phosphoglycerat-Familie, insbesondere von L-Serin und L-Cystein, sind bereits einige Gene/Allele im Stand der Technik bekannt, deren Einsatz zu einer Aminosäure-Überproduktion führen:
- serA-Allele wie beschrieben in EP0620853B1 oder EP0931833A2.
   Diese serA-Allele kodieren für 3-Phosphoglycerat-Dehydrogenasen, die einer verminderten Feedback-Hemmung durch L-Serin unterliegen. Dadurch wird die Bildung von 3-Hydroxypyruvat weitgehend vom Serin-Spiegel der Zelle entkoppelt.
- cysE-Allele wie beschrieben in
   - WO 97/15673 oder
   - Nakamori S. et al., 1998, Appl. Env. Microbiol. 64: 1607-1611 oder
   - Takagi H. et al., 1999, FEBS Lett. 452: 323-327 beschrieben, in einen Mikroorganismenstamm eingebracht werden.
   Diese cysE-Allele kodieren für Serin-O-Acetyl-Transferasen, die einer verminderten Feedback-Hemmung durch L-Cystein unterliegen. Dadurch wird die Bildung von O-Acetyl-L-Serin bzw. L-Cystein weitgehend vom Cystein-Spiegel der Zelle entkoppelt.
- Efflux-Gene wie beschrieben in EP0885962A1
   Das beschriebene orf-Gen kodiert wahrscheinlich für ein Efflux-System, das zur Ausschleusung von Antibiotika und anderern toxischen Stoffen geeignet ist und die Überproduktion von L-Cystein, L-Cystin, N-Acetyl-Serin und/oder Thiazolidinderivaten bewirkt.
- cysB-Gen wie beschrieben in DE19949579C1
   Das cysB-Gen kodiert für einen zentralen Genregulator des Schwefelstoffwechsel und spielt somit eine entscheidende Rolle bei der Bereitstellung von Sulfid für die Cystein-Biosynthese.

Aus dem Stand der Technik ist ebenfalls bekannt, dass die angegebenen Verfahren auch zu Cystein-Derivaten führen können. So kann LL-Cystin als Oxidationsprodukt von L-Cystein oder 2-Methyl-thiazolidin-2,4-dicarbonsäure als Kondensationsprodukt von L-Cystein und Pyruvat während der Fermentation entstehen. Da L-Cystein der zentrale Schwefel-Donor der Zelle ist, können die beschriebenen Verfahren auch als Ausgangspunkt zur Herstellung verschiedenster schwefelhaltiger Metaboliten (z.B. L-Methionin, (+)-Biotin, Thiamin etc.) benützt werden, die im Sinne der vorliegenden Erfindung als L-Cystein-Derivate aufzufassen sind.

Außerdem wurde beschrieben, dass bei geeigneter Vorgehensweise auch die Aminosäuren N-Acetyl-L-Serin (EP-A1-0885962) bzw. O-Acetyl-L-Serin (DE-A-10107002) als Hauptfermentationsprodukte gebildet werden können. L-Serin kann wiederum gemäß DE-A-10219851 relativ einfach aus N-Acetyl-L-Serin-haltigen Fermentationsbrühen gewonnen werden.

EP-A-1 016 710 offenbart in den Beispielen 1 - 8 die Produktion verschiedener Aminosäuren nach Überexpression eines Plasmids, welches das yfik-Gen enthält in E. coli TG1.

NAKAMORI ET AL: 'overproduction of L-cysteine and L-cystine by Escherichia coli strains with a genetically altered serine a-cetyltransferase' APPLIED AND ENVIRONMENTAL MICROBIOLOGY, WASHINGTON,DC, US, Bd. 64, Nr. 5, Mai 1998 (1998-05), Seiten 1607 - 1611, XP002115630 ISSN: 0099-2240 offenbart die Herstellung von L-Cystein und L-Cystin in E. coli durch eine mutierte Serinacetyltransferase.

Aufgabe der vorliegenden Erfindung ist es, einen rekombinanten Mikroorganismenstamm zur Verfügung zu stellen, der eine Überproduktion von O-Acetyl-L-Serin, N-Acetyl-L-Serin oder L-Cystein ermöglicht. Eine weitere Aufgabe ist es, ein fermentatives Verfahren für die Herstellung von O-Acetyl-L-Serin, N-Acetyl-L-Serin oder L-Cystein mittels des rekombinanten Mikroorganismenstammes zur Verfügung zu stellen.

Die erstgenannte Aufgabe wird gelöst durch einen Mikroorganismenstamm, der zur fermentativen Herstellung von O-Acetyl-L-Serin, N-Acetyl-L-Serin oder L-Cystein geeignet ist, enthaltend ein cysE-Allele kodierend für eine Serin-O-Acetyl-Transferase, die eine verminderte Feedback-Hemmung durch L-Cystein aufweist, herstellbar aus einem Ausgangsstamm, dadurch gekennzeichnet, dass er eine gegenüber dem Ausgangsstamm verstärkte Expression des yfiK-Gens oder eines yfiK-Homologs aufweist.

Das yfiK-Gen von Escherichia coli wurde im Rahmen der Genomsequenzierung (Blattner et al. 1997, Science 277:1453-1462) als offener Leserahmen identifiziert und kodiert für ein Protein mit 195 Aminosäuren. Bisher konnte dem yfiK-Gen keine physiologische Funktion zugeordnet werden. Auch eine Datenbankrecherche nach Proteinen mit Sequenzhomologie (FASTA-Algorithmus von GCG Wisconsin Package, Genetics Computer Group (GLG) Madison, Wisconsin) liefert wenig Aufschluß, da lediglich Ähnlichkeiten zu Proteinen angezeigt werden, deren Funktion ebenfalls unbekannt ist. Der einzige Anhaltspunkt für eine mögliche Aktivität des yfiK-Genproduktes ist bei Aleshin et al. (Trends in Biol. Sci., 1999, 24: 133-135) zu finden. Darin wird ein Strukturmotiv postuliert, das eine Proteinfamilie von Aminosäure-Efflux-Proteinen charakterisieren soll. Da dieses schwache Consensus-Motiv auch im YfiK-Protein vorkommt, könnte das YfiK-Protein ein Efflux-System für Aminosäuren darstellen. Es ist jedoch für den Fachmann absolut unmöglich, daraus Schlüsse auf konkrete Aminosäuresubstrate des YfiK-Protein zu ziehen. Der Befund, dass das YfiK-Genprodukt bei der Produktion von Aminosäuren der Phosphoglycerat-Familie einen positiven Beitrag leistet, ist insbesondere deshalb überraschend, da mit dem YdeD-Genprodukt bereits ein Efflux-Protein für Aminosäuren der Phosphoglyceratfamilie in Escherichia coli charakterisiert wurde(Daßler et al. Mol. Microbiol., 2000, 36: 1101-1112) und die Existenz eines zweiten Systems völlig unerwartet ist. Interessanterweise bestehen keine Strukturähnlichkeiten zwischen den yfiK- und ydeD-Genprodukten.

Das yfiK-Gen und das YfiK-Genprodukt (YfiK-Protein) sind durch die Sequenzen SEQ ID No. 1 beziehungsweise SEQ ID No. 2 charakterisiert. Im Rahmen der vorliegenden Erfindung sind als yfiK-Homologe solche Gene aufzufassen, die bei einer Analyse mit dem Algorithmus BESTFIT (GCG Wisconsin Package, Genetics Computer Group (GLG) Madison, Wisconsin) eine Sequenzidentität von größer 30 % aufweisen. Besonders bevorzugt ist eine Sequenzidentität von größer 70 %.

Ebenso sind Proteine mit einer Sequenzidentität von größer 30 % (Algorithmus BESTFIT (GCG Wisconsin Package, Genetics Computer Group (GLG) Madison, Wisconsin) als YfiK-homologe Proteine aufzufassen. Besonders bevorzugt ist eine Sequenzidentität von größer 70 %.

Somit sind als yfiK-Homologe auch Allelvarianten des yfiK-Gens zu verstehen, insbesondere funktionelle Varianten, die sich durch Deletion, Insertion oder Substitution von Nukleotiden aus der in SEQ ID No. 1 dargestellten Sequenz ableiten, wobei die enzymatische Aktivität des jeweiligen Genprodukts jedoch erhalten bleibt.

Erfindungsgemäße Mikroorganismen, die eine gegenüber dem Ausgangsstamm verstärkte Expression des yfiK-Genprodukts aufweisen, können mit Standardtechniken der Molekularbiologie erzeugt werden.

Als Ausgangsstämme sind prinzipiell alle Organismen geeignet, die den Biosyntheseweg für Aminosäuren der Phosphoglycerat-Familie aufweisen, rekombinanten Verfahren zugänglich sind und durch Fermentation kultivierbar sind. Solche Mikroorganismen können Pilze, Hefen oder Bakterien sein. Bevorzugt handelt es sich um Bakterien der phylogenetischen Gruppe der Eubacteria. Besonders bevorzugt um Mikroorganismen der Familie Enterobacteriaceae und insbesondere der Art Escherichia coli.

Die Kopienzahl das yfiK-Gens in einem Mikroorganismus kann erhöht sein und/oder es kann durch geeignete Promotoren die Expression des yfiK-Gens gesteigert sein. Unter verstärkter Expression ist dabei vorzugsweise zu verstehen, daß das yfiK-Gen mindestens doppelt so stark exprimiert wird, wie im Ausgangsstamm.

Die Erhöhung der Kopienzahl des yfiK-Gens in einem Mikroorganismus kann mit dem Fachmann bekannten Methoden vorgenommen werden. So kann zum Beispiel das yfiK-Gen in Plasmid-Vektoren mit mehrfacher Kopienzahl pro Zelle (z.B. pUC19, pBR322, pACYC184 für Escherichia coli) kloniert und in den Mikroorganismus eingebracht werden. Alternativ kann das yfiK-Gen mehrfach ins Chromosom eines Mikroorganismus integriert werden. Als Integrationsverfahren können die bekannten Systeme mit temperenten Bakteriophagen, integrative Plasmide oder die Integration über homologe Rekombination genutzt werden (z.B. Hamilton et al., 1989, J. Bacteriol. 171: 4617-4622).

Bevorzugt ist die Erhöhung der Kopienzahl durch Klonierung eines yfiK-Gens in Plasmid-Vektoren unter der Kontrolle eines Promotors. Besonders bevorzugt ist die Erhöhung der Kopienzahl in Escherichia coli durch Klonierung eines yfiK-Gens in einem pACYC-Derivat wie z. B. pACYC184-LH (hinterlegt gemäß Budapester Vertrag bei der Deutschen Sammlung für Mikroorganismen und Zellkulturen, Braunschweig am 18.8.95 unter der Nummer DSM 10172).

Als Kontrollregion für die Expression eines plasmid-kodierten yfiK-Gens kann die natürliche Promotor- und Operatorregion des Gens dienen.

Die verstärkte Expression eines yfiK-Gens kann jedoch insbesondere auch mittels anderer Promotoren erfolgen. Entsprechende Promotorsysteme wie beispielsweise in Escherichia coli der konstitutive GAPDH-Promotor des gapA-Gens oder die induzierbaren lac-, tac-, trc-, lambda-, ara oder tet-Promotoren sind dem Fachmann bekannt (Makrides S. C., 1996, Microbiol. Rev. 60: 512-538). Solche Konstrukte können in an sich bekannter Weise auf Plasmiden oder chromosomal verwendet werden.

Des weiteren kann eine verstärkte Expression dadurch erreicht werden, daß Translationsstartsignale, wie z. B. die Ribosomenbindestelle oder das Startcodon des Gens, in optimierter Sequenz auf dem jeweiligen Konstrukt vorhanden sind oder daß gemäß der "codon usage" seltene Kodons gegen häufiger vorkommende Kodons ausgetauscht werden.

Mikroorganismenstämme mit den genannten Modifikationen sind bevorzugte Ausführungen der Erfindung.

Die Klonierung eines yfiK-Gens in Plasmid-Vektoren erfolgt beispielsweise durch spezifische Amplifikation mittels der Polymerase-Ketten-Reaktion unter Einsatz von spezifischen Primern, die das komplette yfiK-Gen erfassen, und anschließende Ligation mit Vektor-DNS-Fragmenten.

Als bevorzugte Vektoren für die Klonierung eines yfiK-Gens werden Plasmide verwendet, die bereits Promotoren zur verstärkten Expression enthalten, beispielsweise den konstitutiven GAPDH-Promotor des gapA-Gens von Escherichia coli.

Des weiteren sind Vektoren besonders bevorzugt die bereits ein Gen/Allel enthalten, dessen Einsatz zu einer Überproduktion von Aminosäuren der Phosphoglycerat-Familie führt, wie beispielsweise das cysEX-Gen (WO97/15673). Solche Vektoren ermöglichen die direkte Herstellung von erfindungsgemäßen Mikroorganismenstämmen mit hoher Aminosäure-Überproduktion aus einem beliebigen Mikroorganismenstamm, da ein solches Plasmid auch eine Verminderung der Feedback-Hemmung des Cysteinstoffwechsels in einem Mikroorganismus bewirkt.

Die Erfindung betrifft somit auch ein Plasmid, das dadurch gekennzeichnet ist, daß es ein genetisches Element zur Deregulierung des Cysteinstoffwechsels kodierend eine Serin-O-Acetyl-Transferase, die eine verminderte Feedback-Hemmung durch L-Cystein aufweist und ein yfiK-Gen mit einem Promotor enthält.

Durch eine gängige Transformationsmethode (z.B. Elektroporation) werden die yfiK-haltigen Plasmide in Mikroorganismen eingebracht und beispielsweise mittels Antibiotika-Resistenz auf plasmid-tragende Klone selektiert.

Die Erfindung betrifft somit auch Verfahren zur Herstellung eines erfindungsgemäßen Mikroorganismenstammes, dadurch gekennzeichnet, daß in einen Ausgangsstamm ein erfindungsgemäßes Plasmid eingebracht wird.

Die Produktion der Aminosäuren O-Acetyl-L-Serin, N-Acetyl-L-Serin oder L-Cystein mit Hilfe eines erfindungsgemäßen Mikroorganismenstammes erfolgt in einem Fermenter nach an und für sich bekannten Verfahren.

Die Erfindung betrifft somit auch ein Verfahren zur Herstellung der Aminosäuren O-Acetyl-L-Serin, N-Acetyl-L-Serin oder L-Cystein, welches dadurch gekennzeichnet ist, dass ein erfindungsgemäßer Mikroorganismenstamm in einer Fermentation eingesetzt wird und die produzierte Aminosäure aus dem Fermentationsansatz abgetrennt wird.

Die Anzucht des Mikroorganismenstammes im Fermenter erfolgt als kontinuierliche Kultur, als batch-Kultur oder vorzugsweise als fed-batch-Kultur. Besonders bevorzugt wird eine C-Quelle während der Fermentation kontinuierlich zudosiert.

Als C-Quelle dienen vorzugsweise Zucker, Zuckeralkohole oder organische Säuren. Besonders bevorzugt werden im erfindungsgemäßen Verfahren als C-Quellen Glukose, Laktose oder Glycerin eingesetzt.

Bevorzugt ist die Dosierung der C-Quelle in einer Form, die gewährleistet, dass der Gehalt an C-Quelle im Fermenter während der Fermentation in einem Bereich von 0,1 - 50 g/l gehalten wird. Besonders bevorzugt ist ein Bereich von 0,5 - 10 g/l.

Als N-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Statisierung wird während der Fermentation regelmäßig diese N-Quelle nachdosiert.

Als weitere Medienzusätze können Salze der Elemente Phosphor, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink und Nickel zugesetzt werden.

Des weiteren können organische Säuren (z.B. Acetat, Citrat), Aminosäuren (z.B. Isoleucin) und Vitamine (z.B. B1, B6) dem Medium zugesetzt werden.

Als komplexe Nährstoffquellen können z.B. Hefeextrakt, Maisquellwasser, Sojamehl oder Malzextrakt zum Einsatz kommen.

Die Inkubationstemperatur für mesophile Mikroorganismen beträgt vorzugsweise 15 - 45 °C, besonders bevorzugt 30 - 37 °C.

Die Fermentation wird vorzugsweise unter aeroben Wachstumsbedingungen durchgeführt. Der Sauerstoffeintrag in den Fermenter erfolgt mit Pressluft oder mit reinem Sauerstoff.

Der pH-Wert des Fermentationsmediums liegt während der Fermentation bevorzugt im pH-Bereich von 5,0 bis 8,5, besonders bevorzugt ist ein pH-Wert von 7,0. Ist die erfindungsgemäße Herstellung von O-Acetyl-L-Serin gewünscht, liegt der besonders bevorzugte pH-Bereich zwischen 5,5 und 6,5.

Für die Herstellung von L-Cystein und L-Cystein-Derivaten muß während der Fermentation eine Schwefelquelle zugefüttert werden. Bevorzugt kommen dabei Sulfate oder Thiosulfate zum Einsatz.

Mikroorganismen, die nach dem beschriebenen Verfahren fermentiert werden, sezernieren in einem Batch- oder Fedbatch-Prozess nach einer Anwachsphase in einem Zeitraum von 10 bis 150 Stunden die Aminosäuren O-Acetyl-L-Serin, N-Acetyl-L-Serin oder L-Cystein in hoher Effizienz in das Kulturmedium.

Die folgenden Beispiele dienen der weiteren Erläuterung der Erfindung.

### Beispiel 1: Klonierung des yfiK-Gens

Das yfiK-Gen aus Escherichia coli Stamm W3110 wurde mit Hilfe der Polymerase-Ketten-Reaktion amplifiziert. Als spezifische Primer dienten die Oligonukleotide und

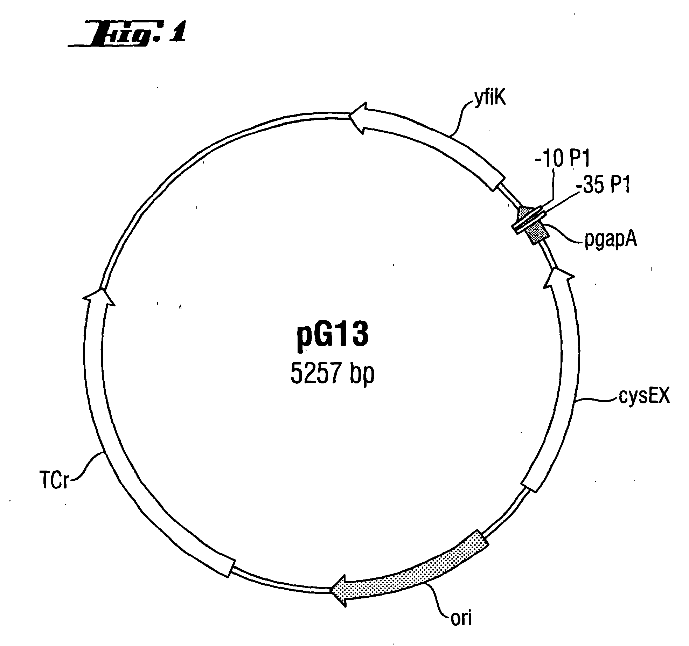

Das resultierende DNS-Fragment wurde mit den Restriktionsenzymen AsnI und PacI verdaut, mit Hilfe einer Agarose-Gelelektrophorese gereinigt und isoliert (Qiaquick Gel Extraction Kit, Qiagen, Hilden, D). Die Klonierung erfolgte durch Ligation mit einem NdeI/PacI-geschnittenen Vektor pACYC184-cysEX-GAPDH, der in EP0885962A1 eingehend beschrieben wurde. Dieser Vektor enthält ein cysEX-Gen, das für eine Serinacetyltransferase mit verminderter Feedback-Hemmung durch L-Cystein kodiert, und 3'-seitig davon den konstitutiven GAPDH-Promoter des gapA-Gens. Durch das angegebene Vorgehen wird das yfiK-Gen so hinter dem GAPDH-Promotor plaziert, dass die Transkription von dort aus initiert werden kann. Der resultierende Vektor trägt die Bezeichnung pG13 und ist in Abbildung 1 als Übersichtszeichnung gezeigt. Nach der Verifizierung des Konstrukts wurde der Escherichia coli Stamm W3110 transformiert und entsprechende Transformanten mit Tetracyclin selektiert. Der Bakterienstamm Escherichia coli W3110 / pG13 wurde bei der DSMZ (Deutsche Sammlung für Mikroorganismen und Zellkulturen GmbH, D-38142 Braunschweig) unter der Nummer DSM 15095 gemäß Budapester Vertrag hinterlegt und in folgenden Beispielen als Produktionstamm zur Herstellung von Aminosäuren der Phosphoglycerat-Familie genützt. Als Vergleichsstamm zur Demonstration des Effektes der erhöhten Expression des yfiK-Gens wurde W3110 /pACYC184-cysEX gewählt, der ebenfalls in EP0885962A1 eingehend beschrieben ist, aber im Unterschied zu pG13 keine GAPDH-Promotor-yfiK-Sequenz enthält.

### Beispiel 2: Vorkultur des Produktionsstammes

Als Vorkultur für die Fermentation wurden 20 ml LB-Medium (10 g/l Trypton, 5 g/l Hefeextrakt, 10 g/l NaCl), das zusätzlich 15 mg/l Tetracyclin enthielt, mit dem Stamm W3110 / pG13 bzw. W3110 / pACYC184-cysEX beimpft und bei 30°C und 150 rpm in einem Schüttler inkubiert. Nach sieben Stunden wurde der gesamte Ansatz in 100 ml SM1-Medium (12 g/l K₂HPO₄; 3 g/l KH₂PO₄; 5 g/l (NH₄)₂SO₄; 0,3 g/l MgSO₄ x 7 H₂O; 0,015 g/l CaCl₂ x 2 H₂O; 0,002 g/l FeSO₄ x 7 H₂O; 1 g/l Na₃Citrat x 2 H₂O; 0,1 g/l NaCl; 1 ml/l Spurenelementlösung bestehend aus 0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O), das mit 5 g/l Glukose; 0,5 mg/l Vitamin B₁ und 15 mg/l Tetracyclin supplementiert wurde, überführt. Die weitere Inkubation erfolgte bei 30 °C für 17 Stunden bei 150 rpm.

### Beispiel 3: Fermentative Herstellung von O-Acetyl-L-Serin

Als Fermenter diente ein Biostat M-Gerät der Firma Braun Biotech (Melsungen, D) mit einem maximalen Kulturvolumen von 2 l. Mit der in Beispiel 2 beschriebenen Vorkultur (optische Dichte bei 600 nm von ca. 3) wurde der Fermenter mit 900 ml SM1-Medium, das mit 15 g/l Glukose, 0,1 g/l Trypton, 0,05 g/l Hefeextrakt, 0,5 mg/l Vitamin B₁ und 15 mg/l Tetracyclin supplementiert wurde, beimpft. Während der Fermentation wurde eine Temperatur von 32 °C eingestellt und der pH-Wert durch Zudosierung von 25 %-igem Ammoniak bei einem Wert von 6,0 konstant gehalten. Die Kultur wurde mit entkeimter Druckluft bei 1,5 vol/vol/min begast und mit einer Rührerdrehzahl von 200 rpm gerührt. Nach Absinken der Sauerstoffsättigung auf einen Wert von 50% wurde die Drehzahl über ein Kontrollgerät bis zu einem Wert von 1200 rpm erhöht, um 50 % Sauerstoffsättigung zu erhalten (Bestimmt mit einer pO₂-Sonde, kalibriert auf 100% Sättigung bei 900 rpm). Sobald der Glukose-Gehalt im Fermenter von anfänglich 15 g/l auf ca. 5-10 g/l abgesunken war, erfolgte eine Zudosierung einer 56 % Glukose-Lösung. Die Fütterung erfolgte mit einer Flußrate von 6-12 ml/h, wobei die Glukosekonzentration im Fermenter zwischen 0,5 - 10 g/l konstant gehalten wurde. Die Glukose-Bestimmung wurde mit dem Glukoseanalysator der Firma YSI (Yellow Springs, Ohio, USA) durchgeführt. Die Fermentationsdauer betrug 28 Stunden. Nach dieser Zeit wurden Proben entnommen und die Zellen durch Zentrifugation vom Kulturmedium abgetrennt. Die resultierenden Kulturüberstände wurden durch reversed phase HPLC an einer LUNA 5 µ C18(2)-Säule (Phenomenex, Aschaffenburg, Deutschland) bei einer Flußrate von 0,5 ml/min analysiert. Als Eluent diente verdünnte Phosphorsäure (0,1 ml konz. Phosphorsäure / 1). Die Tabelle 1 zeigt die erzielten Gehalte der Hauptstoffwechselprodukte im Kulturüberstand. Diese sind O-Acetyl-L-Serin und N-Acetyl-L-Serin, das bei neutralen bis alkalischen Bedingungen zunehmend durch Isomerisierung aus O-Acetyl-L-Serin entsteht.

**Tabelle 1 :**

| **Stamm** | **Aminosäure-Gehalt [g/l]** | |
|---|---|---|
| | O-Acetyl-L-Serin | N-Acetyl-L-Serin |
| W3110pACYC184-cysEX | 1,8 | 1,5 |
| W3110/pG13 (cysEX-yfiK) | 7,4 | 3,0 |

### Beispiel 4: Fermentative Herstellung von N-Acetyl-L-Serin

Für die Herstellung von N-Acetyl-L-Serin wurde genauso wie in Beispiel 2 und 3 verfahren. Lediglich der pH-Wert in der Fermentation wurde auf den Wert 7,0 eingestellt. Dadurch wird die Isomerisierung von O-Acetyl-L-Serin zu N-Acetyl-L-Serin begünstigt und als Hauptprodukt N-Acetyl-L-Serin erhalten. Die Fermentationszeit betrug 48 Stunden.

**Tabelle 2 :**

| **Stamm** | **Aminosäure-Gehalt [g/l]** |
|---|---|
| | N-Acetyl-L-Serin |
| W3110pACYC184-cysEX | 5,8 |
| W3110/pG13 (cysEX-yfiK) | 9,2 |

### Beispiel 5: Fermentative Herstellung von L-Cystein und L-Cystein-Derivaten

Für die Herstellung von L-Cystein wurde genauso wie in Beispiel 2 und 3 verfahren. Lediglich der pH-Wert in der Fermentation wurde auf den Wert 7,0 eingestellt und eine Zufütterung von Thiosulfat vorgenommen. Dabei wurde nach zwei Stunden eine Zudosierung einer 30 % Na-Thiosulfat-Lösung mit einer Rate von 3 ml/h vorgenommen. Die Fermentationszeit betrug 48 Stunden. Die Produktion von L-Cystein wurde colorimetrisch mit dem Test von Gaitonde (Gaitonde, M. K. (1967), Biochem. J. 104, 627-633) verfolgt. Dabei ist zu berücksichtigen, daß der Test nicht zwischen L-Cystein und dem in EP 0885962 A1 beschriebenen Kondensationsprodukt von L-Cystein und Pyruvat (2-Methyl thiazolidin-2,4-dicarbonsäure) diskriminiert. LL-Cystin, das durch Oxidation aus L-Cystein entsteht, wird im Test durch Reduktion mit Dithiothreitol (DTT) in verdünnter Lösung bei pH 8,0 ebenfalls als L-Cystein nachgewiesen.

**Tabelle 3 :**

| **Stamm** | **Aminosäure-Gehalt [g/l]** |
|---|---|
| | L-Cystein + Derivate |
| W3110pACYC184-cysEX | 4,6 |
| W3110/pG13 (cysEX-yfiK) | 7,5 |

## Patentansprüche

1. Mikroorganismenstamm, der zur fermentativen Herstellung von O-Acetyl-L-Serin, N-Acetyl-L-Serin oder L-Cystein geeignet ist, enthaltend ein cysE-Allele kodierend für eine Serin-O-Acetyl-Transferase, die eine verminderte Feedback-Hemmung durch L-Cystein aufweist, herstellbar aus einem Ausgangsstamm, **dadurch gekennzeichnet, dass** er eine gegenüber dem Ausgangsstamm verstärkte Expression des yfiK-Gens oder eines yfiK-Homologs aufweist.

2. Mikroorganismenstamm gemäß Anspruch 1, **dadurch gekennzeichnet, dass** es sich um einen Pilz, eine Hefe oder ein Bakterium, vorzugsweise aus der Familie Enterobacteriaceae, insbesondere bevorzugt der Art Escherichia coli, handelt.

3. Mikroorganismenstamm gemäß Anspruch 1 oder 2, **dadurch gekennzeichnet, dass** die Kopienzahl das yfiK-Gens in dem Mikroorganismus erhöht ist oder die Expression des yfiK-Gens durch Einsatz geeigneter Promotoren oder Translationssignale gesteigert wurde.

4. Mikroorganismenstamm gemäß Anspruch 3, **dadurch gekennzeichnet, dass** der Promotor ausgewählt ist aus der Gruppe konstitutiver GAPDH-Promotor des gapA-Gens, induzierbarer lac-, tac-, trc-, lambda-, ara- und tet-Promotor.

5. Mikroorganismenstamm gemäß einem der Ansprüche 1 bis 4, **dadurch gekennzeichnet, dass** es sich um einen Escherichia coli Stamm handelt, bei dem die verstärkte Expression des yfiK-Gens auf der Erhöhung der Kopienzahl des yfiK-Gens in einem pACYC-Derivat (DSM10172) beruht.

6. Plasmid, **dadurch gekennzeichnet, dass** es ein genetisches Element zur Deregulierung des Cysteinstoffwechsels kodierend eine Serin-O-Acetyl-Transferase, die eine verminderte Feedback-Hemmung durch L-Cystein aufweist und ein yfiK-Gen mit einem Promotor enthält.

7. Verfahren zur Herstellung eines Mikroorganismenstammes gemäß einem der Ansprüche 1 bis 5, **dadurch gekennzeichnet, dass** in einen Ausgangsstamm ein Plasmid gemäß Anspruch 6 eingebracht wird.

8. Verfahren zur Herstellung von O-Acetyl-L-Serin, N-Acetyl-L-Serin oder L-Cystein, **dadurch gekennzeichnet, dass** ein Mikroorganismenstamm gemäß einem der Ansprüche 1 bis 5 in einer Fermentation eingesetzt und die produzierte Aminosäure aus dem Fermentationsansatz abgetrennt wird.

9. Verfahren gemäß Anspruch 8, **dadurch gekennzeichnet, dass** der Mikroorganismenstamm in einem Fermenter als kontinuierliche Kultur, als batch-Kultur oder vorzugsweise als fed-batch-Kultur angezogen wird.

10. Verfahren nach Anspruch 8 oder 9, **dadurch gekennzeichnet, dass** eine C-Quelle während der Fermentation kontinuierlich zudosiert wird.

11. Verfahren nach einem der Ansprüche 8 bis 10, **dadurch gekennzeichnet, dass** als C-Quelle Zucker, Zuckeralkohole oder organische Säuren dienen.

12. Verfahren nach einem der Ansprüche 8 bis 11, **dadurch gekennzeichnet, dass** die Dosierung der C-Quelle in einer Form erfolgt, die gewährleistet, dass der Gehalt an C-Quelle im Fermenter während der Fermentation in einem Bereich von 0,1 - 50 g/l, besonders bevorzugt ist ein Bereich von 0,5 - 10 g/l gehalten wird.

13. Verfahren nach einem der Ansprüche 8 bis 12, **dadurch gekennzeichnet, dass** als N-Quelle Ammoniak, Ammoniumsalze oder Proteinhydrolysate verwendet werden.

14. Verfahren nach einem der Ansprüche 8 bis 13, **dadurch gekennzeichnet, dass** die Fermentation unter aeroben Wachstumsbedingungen erfolgt.

## Claims

1. Microorganism strain suitable for fermentative production of O-acetyl-L-serine, N-acetyl-L-serine or L-cysteine, comprising a cysE allele coding for a serine O-acetyl transferase which has a reduced feedback inhibition by L-cysteine, producible from a starting strain, **characterized in that** it has an expression of the yfiK-gene, or of a yfiK homologue, which is increased compared to said starting strain.

2. Microorganism strain according to Claim 1, **characterized in that** it is a fungus, a yeast or a bacterium, preferably of the family Enterobacteriaceae, particularly preferably of the species Escherichia coli.

3. Microorganism strain according to Claim 1 or 2, **characterized in that** the copy number of the yfiK gene is increased in the microorganism or expression of said yfiK gene was increased by using suitable promoters or translation signals.

4. Microorganism strain according to Claim 3, **characterized in that** the promoter is selected from the group consisting of constitutive GAPDH promoter of the gapA gene, inducible lac, tac, trc, lambda, ara and tet promoters.

5. Microorganism strain according to any of Claims 1 to 4, **characterized in that** it is an Escherichia coli strain in which the increased expression of the yfiK gene is based on the increase in the copy number of the yfiK gene in a pACYC derivative (DSM10172).

6. Plasmid, **characterized in that** it has a genetic element for the deregulation of cysteine metabolism encoding a serine O-acetyl transferase which has a reduced feedback inhibition by L-cysteine and comprises a yfiK gene with a promoter.

7. Method for preparing a microorganism strain according to any of Claims 1 to 5, **characterized in that** a plasmid according to Claim 6 is introduced into a starting strain.

8. Method for preparing O-acetyl-L-serine, N-acetyl-L-serine or L-cysteine, **characterized in that** a microorganism strain according to any of Claims 1 to 5 is used in a fermentation and the amino acid produced is removed from the fermentation mixture.

9. Method according to Claim 9, **characterized in that** the microorganism strain is grown in a fermenter as continuous culture, as batch culture or, preferably, as fed-batch culture.

10. Method according to Claim 8 or 9, **characterized in that** a carbon source is continuously metered in during fermentation.

11. Method according to any of Claims 8 to 10, **characterized in that** the carbon sources used are sugars, sugar alcohols or organic acids.

12. Method according to any of Claims 8 to 11, **characterized in that** the carbon source is metered in in a way so as to ensure that the carbon source content in the fermenter is kept in a range from 0.1 - 50 g/l, particularly preferably in a range from 0.5 - 10 g/l, during fermentation.

13. Method according to any of Claims 8 to 12, **characterized in that** the nitrogen sources used are ammonia, ammonium salts or protein hydrolysates.

14. Method according to any of Claims 8 to 13, **characterized in that** fermentation is carried out under aerobic growth conditions.

## Revendications

1. Souche de micro-organismes qui convient pour produire par fermentation de la O-acétyl-L-sérine, de la N-acétyl-L-sérine ou de la L-cystéine, qui contient un allèle cysE qui code pour une sérine-O-acétyl-transférase, qui présente une moindre inhibition par rétroaction par la L-cystéine, que l'on peut préparer à partir d'une souche de départ, **caractérisée en ce que** le gène yfiK ou un homologue yfiK y sont plus fortement exprimés que dans la souche de départ.

2. Souche de micro-organismes selon la revendication 1, **caractérisée en ce qu'**il s'agit d'un champignon, d'une levure ou d'une bactérie, de préférence de la famille des entérobactériacées et de façon particulièrement préférée du type Escherichia coli.

3. Souche de micro-organismes selon la revendication 1 ou 2, **caractérisée en ce qu'**on accroît le nombre de répliques du gène yfiK dans le micro-organisme ou on renforce l'expression du gène yfiK en utilisant des promoteurs ou des signaux de traduction appropriés.

4. Souche de micro-organismes selon la revendication 3, **caractérisée en ce que** l'on sélectionne le promoteur dans le groupe constitué du promoteur GAPDH du gène gapA et des promoteurs lac, tac, trc, lambda, ara et tet inductibles.

5. Souche de micro-organismes selon l'une quelconque des revendications 1 à 4, **caractérisée en ce qu'**il s'agit d'une souche d'Escherichia coli dans laquelle le renforcement de l'expression du gène yfiK repose sur l'augmentation du nombre de répliques du gène yfiK dans un dérivé de pACYC (DSM 10 172).

6. Plasmide **caractérisé en ce qu'**il contient un élément génétique de dérégulation du métabolisme de la cystéine qui code pour une sérine-O-acétyl-transférase, qui présente une moindre inhibition par rétroaction par la L-cystéine et contient un gène yfiK avec un promoteur.

7. Procédé de préparation d'une souche de micro-organismes selon l'une quelconque des revendications 1 à 5, **caractérisée en ce que** l'on introduit un plasmide selon la revendication 6 dans une souche de départ.

8. Procédé de préparation d'O-acétyl-L-sérine, de N-acétyl-L-sérine ou de L-cystéine, **caractérisé en ce que** l'on fait fermenter une souche de micro-organismes selon l'une quelconque des revendications 1 à 5 et on extrait du mélange de fermentation l'acide aminé produit.

9. Procédé selon la revendication 8, **caractérisé en ce que** l'on cultive la souche de micro-organismes dans un fermenteur, en continu, de manière discontinue ou de préférence de manière discontinue avec alimentation (fed-batch culture).

10. Procédé selon la revendication 8 ou 9, **caractérisé en ce que** lors de la fermentation, on ajoute en continu et de manière dosée une source de C.

11. Procédé selon l'une quelconque des revendications 8 à 10, **caractérisé en ce que** comme source de C, on utilise des glucides, des alcools de glucides ou des acides organiques.

12. Procédé selon l'une quelconque des revendications 8 à 11, **caractérisé en ce que** l'on dose l'addition de la source de C d'une manière qui garantit lors de la fermentation le maintien de la teneur en source de C dans le fermenteur dans une plage de 0,1 à 50 g/l et de façon particulièrement préférée dans une plage de 0, 5 à 10 g/l.

13. Procédé selon l'une quelconque des revendications 8 à 12, **caractérisé en ce que** comme source de N, on utilise de l'ammoniac, des sels d'ammonium ou des hydrolysats de protéines.

14. Procédé selon l'une quelconque des revendications 8 à 13, **caractérisé en ce que** l'on conduit la fermentation dans des conditions aérobies de croissance.


### SEQUENZ PROTOKOLL

<110> Consortium fuer elektrochemische Industrie GmbH
<120> Verfahren zur fermentativen Herstellung von Aminosaeuren und Aminosaeure-Derivaten der Phosphoglycerat-Familie
<130> yfiK
<140>
   <141>
<160> 4
<170> PatentIn Ver. 2.0
<210> 1
   <211> 750
   <212> DNA
   <213> Escherichia coli
<220>
   <221> CDS
   <222> (110)..(694)
<400> 1
   gatccataac cccaaaccta tcgaaaatat cgaatctaga atataaaaac attcattttt 60
   ttaaatgttc cgtgtcgggt actgtctacc aaaacagagg agataacaa gtg aca ccg 118
                                                         Val Thr Pro
                                                           1
   acc ctt tta agt gct ttt tgg act tac acc ctg att acc gct atg acg   166
   Thr Leu Leu Ser Ala Phe Trp Thr Tyr Thr Leu Ile Thr Ala Met Thr
         5                  10                  15
   cca gga ccg aac aat att ctc gcc ctt agc tct gct acg tcg cat gga   214
   Pro Gly Pro Asn Asn Ile Leu Ala Leu Ser Ser Ala Thr Ser His Gly
   20                  25                  30                  35
   ttt cgt caa agt acc cgc gtg ctg gca ggg atg agt ctg gga ttt ttg    262
   Phe Arg Gln Ser Thr Arg Val Leu Ala Gly Met Ser Leu Gly Phe Leu
                   40                  45                  50
   att gtg atg tta ctg tgt gcg ggc att tca ttt tca ctg gca gtg att    310
   Ile Val Met Leu Leu Cys Ala Gly Ile Ser Phe Ser Leu Ala Val Ile
               55                  60                  65
   gac ccg gca gcg gta cac ctt ttg agt tgg gcg ggg gcg gca tat att    358
   Asp Pro Ala Ala Val His Leu Leu Ser Trp Ala Gly Ala Ala Tyr Ile
           70                  75                  80
   gtc tgg ctg gcg tgg aaa atc gcc acc agc cca aca aag gaa gac gga    406
   Val Trp Leu Ala Trp Lys Ile Ala Thr Ser Pro Thr Lys Glu Asp Gly
       85                  90                  95
   ctt cag gca aaa cca atc agc ttt tgg gcc agc ttt gct ttg cag ttt    454
   Leu Gln Ala Lys Pro Ile Ser Phe Trp Ala Ser Phe Ala Leu Gln Phe
   100                 105                 110                 115
   gtg aac gtc aaa atc att ttg tac ggt gtt acg gca ctg tcg acg ttt    502
   Val Asn Val Lys Ile Ile Leu Tyr Gly Val Thr Ala Leu Ser Thr Phe
                   120                 125                 130
   gtt ctg ccg caa aca cag gcg tta agc tgg gta gtt ggc gtc agc gtt    550
   Val Leu Pro Gln Thr Gln Ala Leu Ser Trp Val Val Gly Val Ser Val
               135                 140                 145
   ttg ctg gcg atg att ggg acg ttt ggc aat gtg tgc tgg gcg ctg gcg    598
   Leu Leu Ala Met Ile Gly Thr Phe Gly Asn Val Cys Trp Ala Leu Ala
           150                 155                 160
   ggg cat ctg ttt cag cga ttg ttt cgc cag tat ggt cgc cag tta aat    646
   Gly His Leu Phe Gln Arg Leu Phe Arg Gln Tyr Gly Arg Gln Leu Asn
       165                 170                 175
   atc gtg ctt gcc ctg ttg ctg gtc tat tgc gcg gta cgc att ttc tat 694
   Ile Val Leu Ala Leu Leu Leu Val Tyr Cys Ala Val Arg Ile Phe Tyr
   180 185 190 195
   taacgaaaaa aagcggaaga ggtcgccctc ttccgcttag taacttgcta cttaag 750
<210> 2
   <211> 195
   <212> PRT
   <213> Escherichia coli
<400> 2
   Val Thr Pro Thr Leu Leu Ser Ala Phe Trp Thr Tyr Thr Leu Ile Thr
   1 5 10 15
   Ala Met Thr Pro Gly Pro Asn Asn Ile Leu Ala Leu Ser Ser Ala Thr
   20 25 30
   Ser His Gly Phe Arg Gln Ser Thr Arg Val Leu Ala Gly Met Ser Leu
   35 40 45
   Gly Phe Leu Ile Val Met Leu Leu Cys Ala Gly Ile Ser Phe Ser Leu
   50 55 60
   Ala Val Ile Asp Pro Ala Ala Val His Leu Leu Ser Trp Ala Gly Ala
   65 70 75 80
   Ala Tyr Ile Val Trp Leu Ala Trp Lys Ile Ala Thr Ser Pro Thr Lys
   85 90 95
   Glu Asp Gly Leu Gln Ala Lys Pro Ile Ser Phe Trp Ala Ser Phe Ala
   100 105 110
   Leu Gln Phe Val Asn Val Lys Ile Ile Leu Tyr Gly Val Thr Ala Leu
   115 120 125
   Ser Thr Phe Val Leu Pro Gln Thr Gln Ala Leu Ser Trp Val Val Gly
   130 135 140
   Val Ser Val Leu Leu Ala Met Ile Gly Thr Phe Gly Asn Val Cys Trp
   145 150 155 160
   Ala Leu Ala Gly His Leu Phe Gln Arg Leu Phe Arg Gln Tyr Gly Arg
   165 170 175
   Gln Leu Asn Ile Val Leu Ala Leu Leu Leu Val Tyr Cys Ala Val Arg
   180 185 190
   Ile Phe Tyr
   195
<210> 3
   <211> 35
   <212> DNA
   <213> Artificial Sequence
<220>
   <223> Primer for PCR
<400> 3
   ggaattcatt aatgatccat aaccccaaac ctatc 35
<210> 4
   <211> 33
   <212> DNA
   <213> Artificial Sequence
<220>
   <223> Primer for PCR
<400> 4
   gccttaatta agtagcaagt tactaagcgg aag 33